(19) Europäisches Patentamt
European Patent Office
Office européen des brevets

(11) Veröffentlichungsnummer: **0 005 530**
**B1**

# (12) EUROPÄISCHE PATENTSCHRIFT

(45) Veröffentlichungstag der Patentschrift: **12.05.82**

(51) Int. Cl.³: **C 07 C 149/32,** C 08 F 2/50

(21) Anmeldenummer: **79101469.9**

(22) Anmeldetag: **14.05.79**

(54) **Neue Mercaptophenylketone und deren Verwendung als Initiatoren für die Photopolymerisation äthylenisch ungesättigter Verbindungen.**

(30) Priorität: **23.05.78 CH 5596/78**

(43) Veröffentlichungstag der Anmeldung: **28.11.79 Patentblatt 79/24**

(45) Bekanntmachung des Hinweises auf die Patenterteilung: **12.05.82 Patentblatt 82/19**

(84) Benannte Vertragsstaaten: **DE FR GB**

(56) Entgegenhaltungen:
**DE-A-2 602 419**
**FR-A-2 158 498**
**FR-A-2 268 774**

(73) Patentinhaber: **CIBA-GEIGY AG, Patentabteilung Postfach, CH-4002 Basel (CH)**

(72) Erfinder: **Kirchmayr, Rudolf, Dr., Ettingerstrasse 9, CH-4147 Aesch (CH)**
Erfinder: **Felder, Louis, Dr., Riehenring 5, CH-4058 Basel (CH)**

**EP 0 005 530 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Neue Mercaptophenylketone und deren Verwendung als Initiatoren für die Photopolymerisation äthylenisch ungesättigter Verbindungen

Die Erfindung betrifft neue Mercaptophenylketone und deren Verwendung im Gemisch mit einem organischen Amin, als Initiatoren für die Photopolymerisation äthylenisch ungesättigter Verbindungen oder für die photochemische Versetzung von Polyolefinen.

Es ist bekannt, daß man die Photopolymerisation von äthylenisch ungesättigten Verbindungen durch aromatische Ketone vom Typ des Benzophenons, des Anthrachinons, Xanthons oder Thioxanthons initiieren kann. Es ist weiterhin aus dem US-Patent 3 759 807 bekannt, daß die Initiatorwirkung solcher aromatischer Ketone durch den Zusatz von organischen Aminen beschleunigt werden kann. Da diese Amine allein meist keine Initiatorwirkung besitzen, wirken sie in Kombination mit aromatischen Ketonen als Aktivatoren oder Beschleuniger. Technisch ist dies von großer Wichtigkeit, da die Produktionsgeschwindigkeit von photochemisch gehärteten Überzügen oder Druckfarben in erster Linie von der Polymerisationsgeschwindigkeit der ungesättigten Verbindungen abhängt. In der DE-OS 2 602 419 werden als Photoinitiatoren Gemische von aromatischen Ketonen, die Thioäthergruppen enthalten, und organischen Aminen beschrieben. Diese Gemische haben den bekannten Gemischen gegenüber gewisse Vorteile, so zeichnen sie sich beispielsweise gegenüber Gemischen von Chlorthioxanthon und organischen Aminen durch geringere Vergilbungstendenz aus. Die Thioätherketone der genannten Patentschrift haben die allgemeine Formel

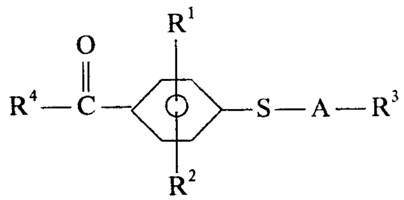

worin $R^4$ ein aromatischer oder heterocyclischer Rest ist, $R^1$ und $R^2$ Wasserstoff oder einwertige Substituenten sind, A ein Alkylenrest und $R^3$ Wasserstoff, Aryl, Benzoyl, Halogen, Alkyl, Alkoxy, Alkylthio, Alkoxyalkyl, Alkaryl, Aralkyl, $NO_2$, CN, COOH oder COOAlkyl sein kann.

Es wurden nunmehr neue aromatische Thioätherketone gefunden, die sich im Gemisch mit organischen Aminen gegenüber den bekannten Mischungen durch bestimmte Vorzüge auszeichnen. Diese Vorzüge können in der höheren Polymerisationsgeschwindigkeit, in der geringeren Vergilbungstendenz bei weiß pigmentierten Beschichtungen, in der besseren Löslichkeit im Substrat oder in der erhöhten Lagerbeständigkeit bestehen.

Die Erfindung betrifft daher Verbindungen der Formel I,

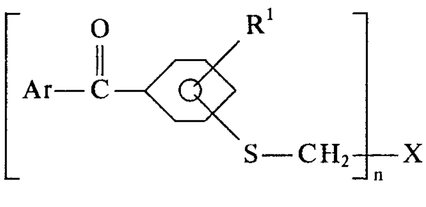

(I)

worin

n 1 oder 2 bedeutet,

Ar einen $C_6-C_{10}$ Arylrest, der unsubstituiert oder durch eine oder mehrere der Gruppen $C_1-C_4$ Alkyl, Phenyl, $C_1-C_4$ Alkoxy, Phenoxy, $C_5-C_6$ Cycloalkoxy, Halogen, $-COOH$, $-COOAlkyl-C_1-C_4$, Benzoyl, oder im Falle von $n=1$ auch durch $-SCH_2X$ substituiert sein kann, oder einen 5- oder 6gliedrigen heteroaromatischen Rest bedeutet,

$R^1$ Wasserstoff, $C_1-C_4$ Alkyl, oder im Falle von $n=1$ auch $-SCH_2X$ bedeutet, und

X im Falle von $n=1$ eine der Gruppen $-CH(R^2)-OH$, $-CH(R^2)-O-CO-R^5$, $-CH_2SH$ oder $-(CH_2)_{1-3}-NR^3R^4$ ist, worin
$R^2$ Wasserstoff, Methyl, Phenyl oder eine der Gruppen $-CH_2OH$, $-CH_2-NR^3R^4$ oder $-CH_2-OR^6$ bedeutet, $R^3$ und $R^4$ unabhängig voneinander Wasserstoff, $C_1-C_8$ Alkyl, oder $C_2-C_4$ Hydroxyalkyl bedeuten oder $R^3$ und $R^4$ zusammen 1,4-Butylen, 1,5-Pentylen oder 3-Oxa-1,5-pentylen bedeuten, $R^5$ $C_1-C_{12}$ Alkyl, $C_2-C_5$ Alkenyl, Phenyl, $C_1-C_{12}$ Alkoxy, Phenoxy, $-NH-Alkyl-C_1-C_{12}$, $-NH$Phenyl oder $-NH$Cyclohexyl bedeutet und
$R^6$ $C_4-C_{12}$ Alkyl oder $C_6-C_{10}$ Aryl bedeutet, und

X im Falle von $n=2$ eine direkte Bindung, $C_1-C_6$ Alkylen, Vinylen, Phenylen oder eine der zweiwertigen Gruppen $-CH_2S-SCH_2-$, $-CH(R^2)-O-CO-O-CH(R^2)-$, $-CH(R^2)-O-CO-R^7-CO-OCH(R^2)-$, $-CO-O-R^8-O-CO-$, $-CH_2COO-R^8-OOCCH_2-$ oder $-CH(OH)-$

$CH_2-O-R^8-O-CH_2-CH(OH)-$ darstellt, worin $R^2$ die oben angegebene Bedeutung hat, $R^7$ $C_2-C_{10}$ Alkylen, Vinylen, Phenylen oder eine Rest $-NH-R^9-NH-$ darstellt, $R^8$ $C_2-C_8$ Alkylen, Phenylen oder $-$Phenylen$-C(CH_3)_2-$Phenylen bedeutet und $R^9$ $C_4-C_{10}$ Alkylen, $C_6-C_{12}$ Arylen, Tolylen oder $-$Phenylen$-CH_2-$Phenylen$-$ bedeutet.

In der genannten Formel I kann Ar ein Phenyl- oder Naphthylrest sein, der durch Substituenten wie z. B. Methyl, Äthyl, Isopropyl, sec.Butyl, Phenyl, Methoxy, Äthoxy, Isopropoxy, Phenoxy, Cyclopentoxy, Cyclohexoxy, F, Cl, Br, $-COOH$, $-COOCH_3$, $-COOC_2H_5$ oder Benzyl substituiert sein kann. Im Falle von n = 1 kann Ar auch ein durch den Rest $-SCH_2X$ substituierter Arylrest sein, wodurch I ein Bis-mercaptoaryl-keton darstellt. Ar kann weiterhin ein heteroaromatischer Rest sein, beispielsweise ein einwertiger Pyrrol-, Thiophen-, Pyridin- oder Furan-Rest.

$R^1$ kann H oder Niederalkyl sein oder im Falle von n = 1 auch $-SCH_2X$. Im letzteren Falle befinden sich am Phenylrest zwei identische Thioäther-Gruppen.

Die Stellung von $R^1$ und $-SCH_2X$ am Phenylring ist beliebig, sie ergibt sich aus der gewählten Synthesemethode. Bevorzugt befindet sich $-SCH_2X$ in 4-Stellung zur Carbonylgruppe. Der am Schwefelatom sitzende Rest $-CH_2X$ ist ein aliphatischer Rest, der durch mindestens eine Hydroxyl-, Äther-, Carbamat-, Ester-, Thiol- oder Aminogruppe substituiert ist. Beispiele für Aminosubstituenten $R^3$ und $R^4$ sind Methyl, Äthyl, Propyl, Butyl, Hexyl, Octyl, 2-Äthylhexyl, 2-Hydroxyäthyl oder 2-Hydroxypropyl. Beispiele für den Ätherrest $R^6$ sind Butyl, Hexyl, Octyl, Dodecyl, Phenyl oder Naphthyl.

Im Falle von n = 2 kann x ein zweiwertiger Alkylenrest sein wie z. B. 1,2-Äthylen, 1,3-Propylen, 1,4-Butylen, 1,6-Hexylen oder 2-Methyl-1,3-propylen. $R^7$ als Alkylen kann ein ebensolcher Rest sein, außerdem auch z. B. Octylen, 2,2-Diäthyl-1,3-propylen oder 1,10-Decylen. $R^8$ und $R^9$ können ebenfalls Alkylenreste sein innerhalb der definierten Kohlenstoff-Anzahl. $R^9$ als Arylen kann z. B. o-Phenylen, p-Phenylen oder 1,4-Naphthylen sein.

Bevorzugt sind Verbindungen der Formel I, worin n = 1 ist, Ar Phenyl, 2-Furyl, 2-Thienyl, 2-Pyridyl oder durch $C_1-C_4$ Alkyl, $C_1-C_4$ Alkoxy, $-COO-C_1-C_4$ Alkyl, $-SCH_2X$ oder Benzoyl substituiertes Phenyl darstellt, $R^1$ Wasserstoff ist und X eine der Gruppen $-CH_2OH$, $-CH(CH_3)-OH$, $-CH(OH)-CH_2-OH$, $-CH_2-NR^3R^4$ oder $-CH-O-CO-R^5$ darstellt, worin $R^3$ und $R^4$ $C_1-C_4$ Alkyl oder Hydroxyäthyl sind und $R^5$ $C_1-C_4$ Alkyl, Phenyl, Vinyl oder Propenyl ist.

Besonders bevorzugt sind Verbindungen der Formel I, worin n = 1, Ar Phenyl, Tolyl oder Xylyl ist, $R^1$ Wasserstoff ist und X $-CH_2OH$, $-CH_2NR^3R^4$ oder $-CH_2O-CO-R^5$ darstellt, worin $R^3$ und $R^4$ Methyl, Äthyl oder zusammen 1,5-Pentylen bedeuten und $R^5$ $C_1-C_4$ Alkyl oder Phenyl bedeutet, insbesondere das 4-(2-Hydroxyäthyl-mercapto)-benzophenon und dessen Carbonsäureester.

Beispiele für Verbindungen der Formel I sind:

4-(2-Hydroxyäthyl-mercapto)-benzophenon
4-(2-Acetoxyäthyl-mercapto)-benzophenon
4-(2-Benzoyloxyäthyl-mercapto)-benzophenon
4-(2-Acryloyloxyäthyl-mercapto)-benzophenon
4-(2-Methoxyäthyl-mercapto)-benzophenon
4-(2-Aminoäthyl-mercapto)-benzophenon
4-(2-Dimethylaminoäthyl-mercapto)-benzophenon
4-(2-Diäthylaminoäthyl-mercapto)-benzophenon
4-(2-Cyclohexylaminoäthyl-mercapto)-benzophenon
4-(3-Aminopropyl-mercapto)-benzophenon
4-(2-Morpholinoäthyl-mercapto)-benzophenon
4-(2-Hydroxyäthyl-mercapto)-4'-methyl-benzophenon
4-(2-Hydroxyäthyl-mercapto)-4'-methoxy-benzophenon
4-(2-Hydroxyäthyl-mercapto)-4'-phenoxy-benzophenon
4-(2-Hydroxyäthyl-mercapto)-2'-carbomethoxy-benzophenon
4-(2-Hydroxyäthyl-mercapto)-2'-carbäthoxy-benzophenon
4-(2,3-Dihydroxypropyl-mercapto)-benzophenon
4-[4-(2-Hydroxyäthyl-mercapto)-benzoyl]-biphenyl
1-[4-(2-Hydroxyäthyl-mercapto)-benzoyl]-naphthalin
2-[4-(2-Hydroxyäthyl-mercapto)-benzoyl]-naphthalin
1,2-Di-[(4-benzoylphenyl)-mercapto]-äthan
1,2-Di-[(4-benzoylphenyl)-mercapto]-xylylen
Di-[2-(4-benzoylphenyl-mercapto)-äthyl]-carbonat.

Die Verbindungen der Formel I sind neue Verbindungen. Sie können auf verschiedene Weise hergestellt werden. Beispielsweise kann man Mercaptophenylketone der Formel II

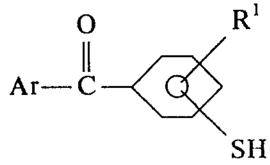

(II)

bzw. deren Alkalisalze mit Halogenalkylverbindungen der Formel $Hal-CH_2-X$ oder $Hal-CH_2-X-CH_2-Hal$ umgesetzt werden, worin Hal Chlor, Brom oder Jod bedeutet und Ar, $R^1$ und X die für Formel I gegebene Bedeutung haben.

Die Verbindungen der Formel II können aber auch mit Epoxiden oder Glycidäthern umgesetzt werden, wobei Verbindungen der Formel I entstehen, worin X $-CH(R^2)OH$ ist. Durch Umsetzung dieser Verbindungen mit gebräuchlichen Acylierungsmitteln, wie z. B. mit Carbonsäurechloriden oder -anhydriden erhält man Verbindungen der Formel I, worin X $-CH_2(R^2)-O-CO-R^5$ oder $-CH(R^2)-OOC-R^7-COO-CH(R^2)-$ ist.

Weiterhin kann man die Verbindungen der Formel II mit Epichlorhydrin umsetzen und das entstandene Chlorhydrin mit primären oder sekundären Aminen, mit Alkalialkoholaten oder mit wäßrigem Alkali in die Verbindungen der Formel I überführen, in denen $R^2$ $-CH_2OH$, $-CH_2NR^3R^4$ oder $-CH_2OR^6$ ist.

Analog verläuft die Umsetzung von Verbindungen der Formel II mit difunktionellen Verbindungen, wie z. B. mit Diglycidyläthern, zu Verbindungen der Formel I, worin $n = 2$ ist.

Ein zweites wichtiges Herstellungsverfahren geht von den aromatischen Halogenketonen der Formel III aus

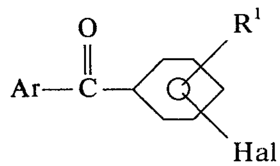

(III)

und setzt diese mit einem Mercaptan der Formel $HS-CH_2X$ oder $HS-CH_2-X-CH_2-SH$ bzw. deren Alkaliverbindungen um. Besonders gut eignen sich dazu die Hydroxyalkylmercaptane $HS-CH_2CH(R^2)-OH$. Die dabei entstehenden Hydroxyl-Verbindungen der Formel I, $X = -CH_2(R^2)OH$, lassen sich in einer Folgereaktion in üblicher Weise mit mono- oder difunktionellen Acylierungsmitteln oder Isocyanaten umsetzen.

Eine weitere Herstellungsmethode ist die Friedel-Crafts-Reaktion mit Komponenten, die bereits die Gruppe $-S-CH_2X$ enthalten, also beispielsweise die Reaktion eines Säurechlorides $Ar-COCl$ mit einer Verbindung der Formel IV

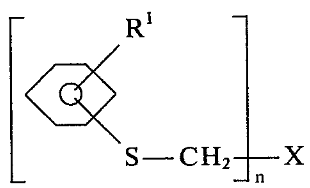

(IV)

in Gegenwart von Aluminiumchlorid.

Gegenstand der Erfindung ist weiterhin die Verwendung eines Gemisches aus

A) einem Mercaptophenylketon der Formel I und
B) einem organischen Amin

als Initiatoren für die Photopolymerisation äthylenisch ungesättigter Verbindungen oder für die photochemische Vernetzung von Polyolefinen.

Die verwendeten organischen Amine können aliphatische, aromatische, araliphatische, cycloaliphatische oder heterocyclische Amine sein. Sie können primäre, sekundäre oder tertiäre Amine sein, bevorzugt sind tertiäre Amine. Beispiele hierfür sind Butylamin, Dibutylamin, Tributylamin, Cyclohexylamin, Benzyl-dimethylamin, Di-cyclohexylamin, Triäthanolamin, N-Methyldiäthanolamin, Phenyl-diäthanolamin, Piperidin, Piperazin, Morpholin, Pyridin, Chinolin, p-Dimethylaminobenzoesäureäthylester, p-Dimethylaminobenzoesäurebutylester, Michlers Keton (4,4'-Bis-dimethylaminobenzophenon) oder 4,4'-Bis-diäthylamino-benzophenon.

Bevorzugt sind Gemische aus

A) einem Mercaptophenylketon der Formel I, worin n 1 ist, Ar Phenyl, 2-Furyl, 2-Thienyl, 2-Pyridyl, mit $C_1-C_4$ Alkyl, $C_1-C_4$ Alkoxy, $-SCH_2X$, $-COOAlkyl-C_1-C_4$ oder Benzoyl substituiertes Phenyl darstellt,
$R^1$ Wasserstoff ist und
X eine der Gruppen $-CH_2OH$, $-CH(CH_3)-OH$, $-CH(OH)-CH_2OH$, $-CH_2-NR^3R^4$ oder $-CH_2O-CO-R^5$ darstellt, worin $R^3$ und $R^4$ $C_1-C_4$ Alkyl oder Hydroxyäthyl sind und $R^5$ $C_1-C_4$ Alkyl, Vinyl oder Propenyl ist, und
B) einem aliphatischen tertiären Amin, einem p-Dimethylaminobenzoesäurealkylester oder Michlers Keton.

Beispiele für aliphatische tertiäre Amine sind Trimethylamin, Triäthylamin, Tri-isopropyl-amin, Tributylamin, Dodecyl-dimethylamin, Octyl-dimethylamin, Triäthanolamin, Tris(hydroxypropyl)amin, N-Methyl-diäthanolamin oder N-Butyl-diäthanolamin.
Besonders bevorzugt sind Gemische aus

A) einem Mercaptophenylketon der Formel I, worin n 1 ist, Ar Phenyl, Tolyl oder Xylyl ist, $R^1$ Wasserstoff ist und X $-CH_2OH$, $-CH_2NR^3R^4$ oder $-CH_2O-CO-R^5$ darstellt, worin $R^3$ und $R^4$ $C_1-C_4$ Alkyl bedeuten oder $R^3$ und $R^4$ zusammen 1,5-Pentylen bedeuten und $R^5$ $C_1-C_4$ Alkyl oder Phenyl bedeutet, und
B) Triäthanolamin oder einem $C_1-C_4$ Alkyl-di-äthanolamin.

Die erfindungsgemäßen Gemische enthalten die Verbindungen der Formel I und die organischen Amine in einem Gewichtsverhältnis von 4 : 1 bis 1 : 4.
Die Gemische werden erfindungsgemäß als Initiatoren für die Photopolymerisation von äthylenisch ungesättigten Verbindungen oder von Systemen, die solche Verbindungen enthalten, verwendet.
Solche photopolymerisierbare Verbindungen sind beispielsweise ungesättigte Monomere wie Ester von Acryl- oder Methacrylsäure, z. B. Methyl-, Äthyl-, n- oder tert.-Butyl-, Isooctyl- oder Hydroxyäthylacrylat, Methyl- oder Äthylmethacrylat, Äthylen-diacrylat, Butandioldiacrylat, Hexandioldiacrylat, Neopentyl-diacrylat, Trimethylolpropan-trisacrylat, Pentaerythrit-tetraacrylat oder Pentaerythrit-trisacrylat; Acrylnitril, Methacrylnitril, Acrylamid, Methacrylamid, N-substituierte (Meth)-acrylamide; Vinylester wie z. B. Vinyl-acetat, -propionat, -acrylat oder -succinat; sonstige Vinylverbindungen wie Vinyläther, Vinylketone, Vinylsulfone, Styrol, Alkylstyrole, Halogenstyrole, Divinylbenzol, N,N'-Divinylharnstoff, Vinylnaphthalin, N-Vinylpyrrolidon, Vinylchlorid oder Vinylidenchlorid; Allylverbindungen wie Diallylphthalat, Diallylmaleat, Triallylisocyanurat, Triallylphosphat oder Äthylenglycol-diallyläther und die Mischungen von solchen ungesättigten Monomeren.
Photopolymerisierbare Verbindungen sind weiterhin ungesättigte Oligomere oder Polymere und deren Mischungen mit ungesättigten Monomeren. Hierzu zählen beispielsweise ungesättigte Polyester, hergestellt aus Phthalsäure und Maleinsäure oder Fumarsäure mit Diolen wie Äthylenglykol, Propylenglykol, Butandiol oder Neopentylglykol, sowie deren Gemische mit Styrol.
Weitere Beispiele sind ungesättigte Acrylharze. Hierzu zählen beispielsweise Umsetzungsprodukte von Polyepoxiden (Epoxiharzen) mit Acrylsäure oder Methacrylsäure oder Umsetzungsprodukte von Polyisocyanaten mit Hydroxyalkylacrylaten sowie die Umsetzungsprodukte von hydroxylgruppenhaltigen Polyestern oder Polyäthern mit Acryl- oder Methacrylsäure. Diese ungesättigten Acrylharze werden meist im Gemisch mit einem oder mehreren Acrylaten eines Mono-, Di- oder Polyalkohols, wie z. B. Äthyl-, Butyl-, Benzyl-, 2-Äthylhexyl- oder 2-Hydroxypropylacrylat, Äthylenglykoldiacrylat, Propylenglykol-diacrylat, Butandiol-diacrylat, Hexamethylen-diacrylat, Trimethylolpropan-trisacrylat oder Pentaerythrit-tetracrylat, verwendet.
Photopolymerisierbare Systeme, wie sie für die verschiedenen Zwecke verwendet werden, enthalten meist außer den photopolymerisierbaren Verbindungen und dem Photoinitiator eine Reihe sonstiger Zusätze. So ist es vielfach üblich, thermische Inhibitoren zuzusetzen, die vor allem während der Herstellung der Systeme durch Mischen der Komponenten vor einer vorzeitigen Polymerisation schützen sollen. Hierzu werden beispielsweise Hydrochinon, Hydrochinonderivate, p-Methoxyphenol oder $\beta$-Naphthole verwendet. Weiter können geringe Mengen von UV-Absorbern zugesetzt werden, wie z. B. solche von Benztriazol- oder Benzophenontyp.
Photopolymerisierbare Systeme enthalten weiterhin — je nach Verwendungszweck — Füllstoffe wie Kieselsäure, Talkum oder Gips, Pigmente, Farbstoffe, Thixotropiemittel oder Verlaufhilfsmittel, wie Silikonöl. Ebenso können photopolymerisierbare Systeme geringe Mengen an Lösungsmittel enthalten.
Ferner können auch Kombinationen mit bekannten Photoinitiatoren, die durch Photofragmentierung Radikale bilden, wie z. B. Benzoinäther, Dialkoxyacetophenone oder Benzilketale, verwendet werden.
Große Bedeutung haben die erfindungsgemäßen Initiatorgemische für die Photohärtung von Druckfarben, da die Trocknungszeit des Bindemittels ein maßgeblicher Faktor für die Produktionsgeschwindigkeit graphischer Erzeugnisse ist und in der Größenordnung von Bruchteilen von Sekunden liegen soll. Gut geeignet sind die erfindungsgemäßen Initiatoren auch für photohärtbare Systeme zur Herstellung von Druckplatten.

Ein weiteres Einsatzgebiet ist die UV-Härtung von Metallbeschichtungen, beispielsweise bei der Lackierung von Blechen für Tuben, Dosen oder Flaschenverschlüssen, sowie die UV-Härtung von Kunststoffbeschichtungen, beispielsweise von Fußböden- oder Wandbelägen auf PVC-Basis.

Beispiele für die UV-Härtung von Papierbeschichtungen sind die farblose Lackierung von Ettiketten, Schallplatten-Hüllen oder Buchumschlägen.

Die erfindungsgemäßen Gemische können auch als Initiatoren zur photochemischen Vernetzung von Polyolefinen verwendet werden. Hierfür kommen z. B. Polypropylen, Polybuten, Polyisobutylen sowie Copolymerisate wie z. B. Äthylen-Propylen-Copolymere in Frage, vorzugsweise jedoch Polyäthylen von niedriger, mittlerer oder hoher Dichte.

Die erfindungsgemäßen Initiator-Gemische werden für die angeführten Anwendungsgebiete zweckmäßig in Mengen von 0,5 bis 20 Gew.-%, vorzugsweise etwa 1 bis 5 Gew.-%, bezogen auf das photopolymerisierbare bzw. vernetzbare System, angewendet. Unter System ist hierbei das Gemisch aus der photopolymerisierbaren bzw. vernetzbaren Verbindung, dem Photoinitiator und den sonstigen Füll- und Zusatzstoffen gemeint wie es in der jeweiligen Anwendung verwendet wird.

Der Zusatz der Photoinitiatoren zu den photopolymerisierbaren Systemen geschieht im allgemeinen durch einfaches Einrühren, da die meisten dieser Systeme flüssig oder gut löslich sind. Meist kommt es zu einer Lösung der erfindungsgemäßen Initiatoren, wodurch deren gleichmäßige Verteilung sowie die Transparenz der Polymerisate gewährleistet ist.

Die Polymerisation erfolgt nach den bekannten Methoden der Photopolymerisation durch Bestrahlung mit Licht, das reich an kurzwelliger Strahlung ist. Als Lichtquellen sind z. B. Quecksilbermitteldruck-, -hochdruck- und -niederdruckstrahler, sowie superaktinische Leuchtstoffröhren geeignet, deren Emissionsmaxima im Bereich zwischen 250 und 400 μm liegen.

Bei der photochemischen Vernetzung von Polyolefinen wird der Photoinitiator dem Polyolefin vor oder während der formgebenden Verarbeitung zugesetzt, beispielsweise durch pulverförmiges Vermischen oder durch Mischen mit dem plastifizierten Polyolefin. Die Vernetzung erfolgt durch Bestrahlung des geformten Gegenstandes in fester Form, beispielsweise in Form von Folien oder Fasern.

Die folgenden Beispiele beschreiben die Herstellung von Verbindungen der Formel I sowie ihre Gemische mit organischen Aminen in ihrer Anwendung als Photoinitiatoren. Hierin bedeuten Teile Gewichtsteile und Prozente Gewichtsprozente. Die Temperaturen sind in °C angegeben.

## Beispiel 1

### 4-(2-Hydroxyäthyl-mercapto)-benzophenon

a) 17,2 g (0,225 Mol) 2-Mercaptoäthanol und 15 g (0,225 Mol) KOH (Gehalt 85%) werden in 200 ml Toluol unter Stickstoff am Wasserabscheider 1 1/2 Stunden unter Rückfluß gehalten. Das abgeschiedene Wasser wird verworfen. Das Toluol wird abdestilliert. Zu erstarrten Kaliumsalz werden 48,8 g (0,225 Mol) 4-Chlorbenzophenon und 150 ml Dimethylacetamid zugegeben. Bei 100°C wird 5 Stunden gerührt. Das Reaktionsgemisch wird auf Wasser gegossen und mit Essigsäure neutralisiert. Das Produkt wird in Äther aufgenommen. Die Ätherschicht wird mit Wasser gewaschen, über $Na_2SO_4$ getrocknet und eingeengt. Das anfallende Produkt wird mit Hexan aufgekocht, die Kristalle werden gesammelt. Ausbeute: 44 g (76%). Smp. 49—52°.

$C_{15}H_{14}O_2S$ (258,34)

| | | | |
|---|---|---|---|
| Berechnet: | C 69,74 | H 5,46 | S 12,41% |
| gefunden: | C 69,9 | H 5,8 | S 12,7% |

b) 11,2 g (0,05 Mol) 4-Chlorbenzophenon, 4,3 g (0,055 Mol) 2-Mercaptoäthanol, 13,8 g (0,1 Mol) $K_2CO_3$ calc. und 15 ml Äthyl-methylketon werden unter Stickstoff 16 Stunden erwärmt (Rückfluß). Das erkaltete Reaktionsgemisch wird mit 50 ml Wasser übergossen und mit HCl angesäuert. Das Produkt wird in Äther aufgenommen. Die Ätherschicht wird mit Wasser gewaschen, über $Na_2SO_4$ getrocknet und eingeengt. Das auch hier als Öl anfallende Produkt kristallisiert beim Stehen. Es wird wie unter a) beschrieben in Hexan aufgekocht. Ausbeute: 10,1 g (77%). Smp. 49—51°.

## Beispiele 2—4

### Ester des 4-(2-Hydroxyäthyl-mercapto)-benzophenons

Allgemeine Vorschrift:

Verestert wird jeweils mit dem entsprechenden Säurechlorid in Tetrahydrofuran unter Zusatz von 1 Äquivalent Diisopropyläthylamin. Nach Beendigung der Reaktion wird das Tetrahydrofuran entfernt. Der Rückstand wird mit Wasser übergossen und mit Äther ausgezogen. Die Ätherschicht wird mit

Wasser gewaschen, über $Na_2SO_4$ getrocknet und eingeengt. Die erhaltenen Rohprodukte werden jeweils über eine Kieselgel-Trockensäure gereinigt (Fließmittel: Essigester/Hexan 1 : 4 oder $CH_2Cl_2$). Ausbeute: jeweils 71—74%.

2) 4-(2-Acetoxyäthyl-mercapto)-benzophenon: Smp. 36—39°

$C_{17}H_{16}O_3S$ (300,37)

| | | | |
|---|---|---|---|
| Berechnet: | C 67,98 | H 5,37 | S 10,68% |
| Gefunden: | C 67,9 | H 5,5 | S 10,6% |

3) 4-(2-Benzoyloxyäthyl-mercapto)-benzophenon: Öl

$C_{22}H_{18}O_3S$ (362,44)

| | | | |
|---|---|---|---|
| Berechnet: | C 72,91 | H 5,01 | S 8,85% |
| Gefunden: | C 73,0 | H 5,1 | S 8,5% |

4) 4-(2-Acryloxyäthyl-mercapto)-benzophenon: Öl

$C_{18}H_{16}O_3S$ (312,38)

| | | | |
|---|---|---|---|
| Berechnet: | C 69,21 | H 5,16 | S 10,27% |
| Gefunden: | C 68,9 | H 5,4 | S 10,4%. |

Beispiel 5

Eine blaue Druckfarbe wird nach folgender Rezeptur hergestellt:

| | |
|---|---|
| 55,0 Gew.-Teile | Setalin AP 560 (Acrylharz der Firma Synthese, Holland) |
| 20,0 Gew.-Teile | Irgalith GLSM (blaues Pigment der Firma Ciba-Geigy) |
| 4,0 Gew.-Teile | Photoinitiator der Formel I |
| 4,0 Gew.-Teile | N-Methyldiäthanolamin |
| 17,0 Gew.-Teile | Ebecryl 150 (Acrylharz der Fa. UCB-Belgien). |

Aus dem Setalin AP 560 und dem Irgalith GLSM wird in einer Kugelmühle eine blaue Farbpaste bereitet.

Der Photohärter wird mit dem Amin in Ebecryl 150 vorgelöst und in die blaue Farbpaste durch Dispergieren auf einer Tellerreibmaschine eingearbeitet. Hierauf wird die Druckfarbe mit einer Auflage von 2 $g/m^2$ mit Hilfe eines Prüfbau-Probedruckgerätes auf Spezialpapier aufgebracht.

Druckbedingungen:

| | |
|---|---|
| Anpreßdruck: | 245 $N/cm^2$ (25 $Kp/cm^2$) |
| Druckgeschwindigkeit: | 2 m/sec. |

Die Proben werden sofort nach dem Andruck in einem Durchgang durch Bestrahlung in einem UV-Gerät (Hersteller: Radiation Polymer Company USA) bei variabler Transportgeschwindigkeit ausgehärtet

Gerätebedingungen:

| | |
|---|---|
| Lampenleistung | 80 W/cm (Standard Quecksilber Dampflampe) |
| Lampenabstand | 11 cm. |

Zur Beurteilung der Aushärtung wird der Abschmiertest herangezogen. Hierbei wird die Geschwindigkeit ermittelt, bei der unter einem Anpreßdruck von 25 $Kp/cm^2$ keine Übertragung der Druckfarbe auf neutrales Papier mehr festgestellt werden kann. Ebenso wird der Abriebtest mit einem REL-SCRATCH-HARDNESS-RECORDER gemäß Defense Specification DES-1053 Method No. 8 durchgeführt. In der folgenden Tabelle sind die gefundenen Meßwerte mit den erfindungsgemäßen Benzophenon-Derivaten zusammengestellt. Die Zahlen in der zweiten Spalte bedeuten dabei die Druck-Geschwindigkeit in m/sec, die möglich ist, um im Abschmiertest keine Übertragung der Druckfarbe zu ergeben. Je höher diese Geschwindigkeit ist, desto rascher härtet die Druckfarbe. Die Zahlen in der dritten Spalte geben die Geschwindigkeit an, nach der der Abriebtest bestanden wird.

Druck- bzw. Härtungsgeschwindigkeit in m/sec

| Verwendeter Initiator | Abschmiertest | REL-Test |
|---|---|---|
| Verbindung Nr. 1 | 2,00 | 1,25 |
| Verbindung Nr. 2 | 1,5 | 1,25 |
| Verbindung Nr. 3 | 1,5 | 1,0 |
| Verbindung Nr. 4 | 1,75 | 1,0 |

Beispiele 6—13

4'-(2-Hydroxyäthyl-mercapto)-3,4-dimethyl-benzophenon

12,2 g (0,05 Mol) 4'-Chlor-3,4-dimethylbenzophenon und 4,3 g (0,055 Mol) 2-Mercaptoäthanol werden in 50 ml Dimethylacetamid unter Stickstoff auf 95° geheizt. Dann werden 13,8 g (0,1 Mol) $K_2CO_3$ calc. zugegeben. Die Suspension wird 6 Stunden bei 90—100°C gehalten. Das erhaltene Reaktionsgemisch wird mit 100 ml Wasser übergossen und mit Äther extrahiert. Die Ätherschicht wird mit Wasser gewaschen, über $Na_2SO_4$ getrocknet und eingeengt. Die anfallenden Kristalle werden aus Methanol umkristallisiert und im Vacuum bei 60°C getrocknet. Ausbeute: 10,1 g (71%). Smp. 92—93°C.

$C_{17}H_{18}O_2S$ (286,39)

| | | | |
|---|---|---|---|
| Berechnet: | C 71,30 | H 6,34 | S 11,19% |
| Gefunden: | C 71,4 | H 6,3 | S 11,1% |

In gleicher Weise werden weitere Benzophenonderivate hergestellt. Die erhaltenen Rohprodukte werden in einem geeigneten Lösungsmittel umkristallisiert und teilweise noch über eine Kieselgel-Trockensäule gereinigt (Fließmittel: Chloroform/Äthanol 95 : 5).

Tabelle 1

Verbindungen der Formel 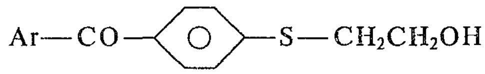

| Bsp. Nr. | Ar | Smp. | Analyse (ber./gef.) C | H | S |
|---|---|---|---|---|---|
| 6 | 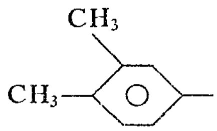 | 92–93° | 71,3/71,4 | 6,34/6,3 | 11,10/11,1 |
| 7 | 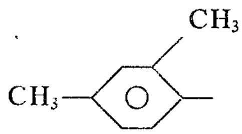 | 64–66° (Ligroin) | 73,1/71,4 | 6,34/6,2 | 11,19/11,1 |
| 8 | 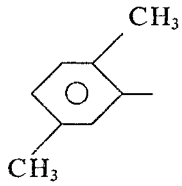 | Öl | 71,3/71,1 | 6,34/6,4 | 11,19/10,5 |
| 9 | 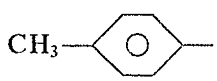 | 99° (Acetonitril) | 70,56/70,7 | 5,92/6,0 | 11,77/11,6 |
| 10 | 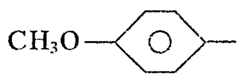 | 102° (Äthanol) | 66,65/66,2 | 5,59/5,6 | 11,12/11,0 |
| 11 | 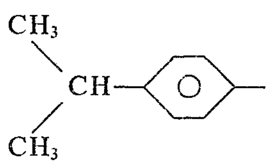 | 56° | 71,97/71,7 | 6,71/6,7 | 10,47/10,5 |
| 12 | 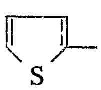 | 65° | 59,06/58,6 | 4,58/4,5 | 24,26/24,3 |
| 13 | 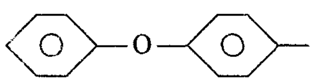 | 122–124° | 71,98/71,6 | 5,18/5,1 | 9,15/9,1 |

### Beispiele 14—17

### 4-(2-N,N-Dimethylaminoäthyl-mercapto)-benzophenon

12,85 g (0,064 Mol) 4-Chlorbenzophenon, 13,6 g (0,13 Mol) 2-N,N-Dimethylaminoäthanthiol und 27,6 g (0,2 Mol) Kaliumcarbonat werden in 100 ml N,N-Dimethylacetamid unter $N_2$-Gas und Rühren 12 Stunden bei 100°C gehalten. Nach dem Abkühlen wird das Reaktionsgemisch in 200 ml Wasser gegossen. Das sich abscheidende Öl wird in Äther aufgenommen. Das Rohprodukt wird über einen Salzsäureauszug und Kugelrohrdestillation (Bad 200—220°C/0,01 mm Hg) gereinigt. Erhalten wird 10,2 g (59%) als Öl anfallendes Produkt.

In analoger Weise wurden erhalten:

15) 2-(2-Hydroxyäthyl-mercapto)-4'-methyl-benzophenon. Viskoses Öl. Analyse (ber./gef.): C 70,56/69,5%, H 5,92/6,2%, O 11,75/11,7%, S 11,77/12,5%.

16) 2-(2-Hydroxyäthyl-mercapto)-benzophenon. Öl. Analyse (ber./gef.): C 69,74/68,9%, H 5,46/5,5%, S 12,41/13,5%.

17) 4-(2,3-Dihydroxypropyl-mercapto)-benzophenon. Smp. 86—88°.

Beispiel 18

Eine blaue Druckfarbe wird nach folgender Rezeptur hergestellt:

165 Teile Setalin AP 560 (Acrylharz der Firma Synthese, Holland)
45 Teile Farbenruß 2/O (Gasruß der Firma Degussa, Frankfurt/Main)
15 Teile Vossenblau 360 (blaues Pigment der Firma Degussa Frankfurt/Main)
27 Teile Ebecryl 150 (Acrylharz der Firma UCB-Belgien).

Diese Komponenten werden angeteigt und dreimal auf einem Dreiwalzenstuhl vermischt. Es werden

4,6 Teile blaue Druckfarbe nach obiger Rezeptur,
0,2 Teile Photoinitiator und
0,2 Teile Triäthanolamin

zusammengegeben und auf einer Tellerreibmaschine homogenisiert. Hierauf wird die Druckfarbe mit einer Auflage von 1,5 g/m² mit Hilfe eines Prüfbau-Probedruckgerätes auf Spezialpapier aufgebracht.

Druckbedingungen:

| | |
|---|---|
| Anpreßdruck | 980,6 N/cm² (100 Kp/cm²) |
| Auftragegeschwindigkeit | 1 m/sec. |

Die Proben werden sofort nach dem Andruck in einem Durchgang durch Bestrahlung in einem UV-Gerät (Hersteller: Radiation Polymer Company USA) bei variabler Transportgeschwindigkeit ausgehärtet.

Gerätebedingungen:

| | |
|---|---|
| Lampenleistung | 80 W/cm (Standard Quecksilber Dampflampe) |
| Lampenabstand | 11 cm |

Zur Beurteilung der Aushärtung wird der Abschmiertest herangezogen. Hierbei wird die Geschwindigkeit ermittelt, bei der unter einem Anpreßdruck von 980 N/cm² (100 Kp/cm²) keine Übertragung der Druckfarbe auf neutrales Papier mehr festgestellt werden kann. Ebenso wird der Abriebtest mit einem REL-SCRATCH-HARDNESS-RECORDER gemäß Defense Spezification DES-1053 Method No. 8 durchgeführt. In der folgenden Tabelle sind die gefundenen Meßwerte mit den erfindungsgemäßen Benzophenon-Derivaten zusammengestellt. Die Zahlen in der zweiten Spalte bedeuten dabei die maximale Druck-Geschwindigkeit in m/s, die möglich ist, um im Abschmiertest keine Übertragung der Druckfarbe zu ergeben. Je höher diese Geschwindigkeit ist, desto rascher härtet die Druckfarbe. Die Zahlen in der dritten Spalte geben die Geschwindigkeit an, nach der der Abriebtest bestanden wird.

Tabelle 2

| Verwendeter Initiator | Abschmiertest | REL-Test |
|---|---|---|
| | Druck- bzw. Härtungsgeschwindigkeit in m/s | |
| Benzophenon | 15 | 5 |
| Verbindung Nr. 1 | 45 | 15 |
| Verbindung Nr. 6 | 45 | 15 |
| Verbindung Nr. 7 | 45 | 15 |
| Verbindung Nr. 9 | 45 | 15 |
| Verbindung Nr. 10 | 45 | 30 |
| Verbindung Nr. 11 | 30 | 30 |
| Verbindung Nr. 13 | 30 | 15 |

**Patentansprüche**

1. Eine Verbindung der Formel I,

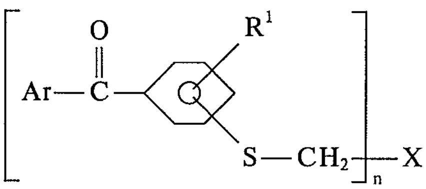

(I)

worin

n 1 oder 2 bedeutet,

Ar einen $C_6-C_{10}$ Arylrest, der unsubstituiert oder durch eine oder mehrere Gruppen $C_1-C_4$ Alkyl, Phenyl, $C_1-C_4$ Alkoxy, Phenoxy, $C_5-C_6$ Cycloalkoxy, Halogen, $-COOH$, $-COOAlkyl-C_1-C_4$, Benzoyl, oder im Falle von n=1 auch durch $-SCH_2X$ substituiert sein kann, oder einen 5- oder 6gliedrigen heteroaromatischen Rest bedeutet,

$R^1$ Wasserstoff, $C_1-C_4$ Alkyl, oder im Falle von n = 1 auch $-SCH_2X$ bedeutet, und

X im Falle von n=1 eine der Gruppen $-CH(R^2)-OH$, $-CH(R^2)-O-CO-R^5$, $-CH_2SH$ oder $-(CH_2)_{1-3}-NR^3R^4$ ist, worin
$R^2$ Wasserstoff, Methyl, Phenyl oder eine der Gruppen $-CH_2OH$, $-CH_2NR^3R^4$ oder $-CH_2-OR^6$ bedeutet, $R^3$ und $R^4$ unabhängig voneinander Wasserstoff, $C_1-C_8$ Alkyl, oder $C_2-C_4$ Hydroxyalkyl bedeuten oder $R^3$ und $R^4$ zusammen 1,4-Butylen, 1,5-Pentylen oder 3-Oxa-1,5-pentylen bedeuten, $R^5$ $C_1-C_{12}$ Alkyl, $C_2-C_5$ Alkenyl, Phenyl, $C_1-C_{12}$ Alkoxy, Phenoxy, $-NH-Alkyl-C_1-C_{12}$, $-NH$Phenyl oder $-NH$Cyclohexyl bedeutet und
$R^6$ $C_4-C_{12}$ Alkyl oder $C_6-C_{10}$ Aryl bedeutet und

X im Falle von n=2 eine direkte Bindung, $C_1-C_6$ Alkylen, Vinylen, Phenylen oder eine der zweiwertigen Gruppen $-CH_2S-SCH_2-$, $-CH(R^2)-O-CO-O-CH(R^2)-$, $-CH(R^2)-O-CO-R^7-CO-OCH(R^2)-$, $-CO-O-R^8-O-CO-$, $-CH_2COO-R^8-OOCCH_2-$ oder $-CH(OH)-CH_2-O-R^8-O-CH_2-CH(OH)-$ darstellt, worin $R^2$ die oben angegebene Bedeutung hat, $R^7$ $C_2-C_{10}$ Alkylen, Vinylen, Phenylen oder einen Rest $-NH-R^9-NH-$ darstellt, $R^8$ $C_2-C_8$ Alkylen, Phenylen oder $-$Phenylen$-C(CH_3)_2-$Phenylen bedeutet und $R^9$ $C_4-C_{10}$ Alkylen, $C_6-C_{12}$ Arylen, Tolylen oder $-$Phenylen$-CH_2-$Phenylen$-$ bedeutet.

2. Eine Verbindung gemäß Anspruch 1 der Formel I, worin
n 1 ist, Ar Phenyl, 2-Furyl, 2-Thienyl, 2-Pyridyl oder durch $C_1-C_4$ Alkyl, $C_1-C_4$ Alkoxy, $-COO-C_1-C_4$ Alkyl, $-SCH_2X$ oder Benzoyl substituiertes Phenyl darstellt, $R^1$ Wasserstoff ist und
X eine der Gruppen $-CH_2OH$, $-CH(CH_3)-OH$, $-CH(OH)-CH_2OH$, $-CH_2-NR^3R^4$ oder $-CH-O-CO-R^5$ darstellt, worin $R^3$ und $R^4$ $C_1-C_4$ Alkyl oder Hydroxyäthyl sind und $R^5$ $C_1-C_4$ Alkyl, Phenyl, Vinyl oder Propenyl ist.

3. Eine Verbindung gemäß Anspruch 1 der Formel I, worin
n=1 ist, Ar Phenyl, Tolyl oder Xylyl ist, $R^1$ Wasserstoff ist und X $-CH_2OH$, $-CH_2NR^3R^4$ oder $-CH_2O-CO-R^5$ darstellt, worin $R^3$ und $R^4$ Methyl, Äthyl oder zusammen 1,5-Pentylen bedeuten und $R^5$ $C_1-C_4$ Alkyl oder Phenyl bedeutet.

4. 4-(2-Hydroxyäthyl-mercapto)-benzophenon oder ein Carbonsäureester davon als Verbindung gemäß Anspruch 1.

5. Verwendung eines Gemisches von

A) einem Mercaptophenylketon der Formel I,

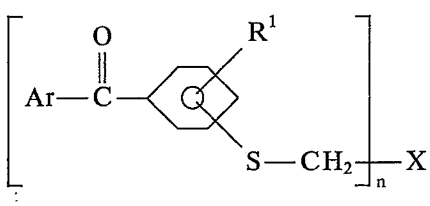

(I)

worin

n 1 oder 2 bedeutet,

Ar einen $C_6-C_{10}$ Arylrest, der unsubstituiert oder durch eine oder mehrere der Gruppen $C_1-C_4$ Alkyl, Phenyl, $C_1-C_4$ Alkoxy, Phenoxy, $C_5-C_6$ Cycloalkoxy, Halogen, $-COOH$, $-COOAlkyl-$

$C_1-C_4$, Benzoyl oder im Falle von n = 1 auch durch $-SCH_2X$ substituiert sein kann, oder einen 5- oder 6gliedrigen heteroaromatischen Rest bedeutet,

$R^1$ Wasserstoff, $C_1-C_4$ Alkyl, oder im Falle von n = 1 auch $-SCH_2X$ bedeutet, und

X im Falle von n = 1 eine der Gruppen $-CH(R^2)-OH$, $-CH(R^2)-O-CO-R^5$, $-CH_2SH$ oder $-(CH_2)_{1-3}-NR^3R^4$ ist, worin

$R^2$ Wasserstoff, Methyl, Phenyl oder eine der Gruppen $-CH_2OH$, $-CH_2-NR^3R^4$ oder $-CH_2-OR^6$ bedeutet, $R^3$ und $R^4$ unabhängig voneinander Wasserstoff, $C_1-C_8$ Alkyl, oder $C_2-C_4$ Hydroxyalkyl bedeuten oder $R^3$ und $R^4$ zusammen 1,4-Butylen, 1,5-Pentylen oder 3-Oxa-1,5-pentylen bedeuten, $R^5$ $C_1-C_{12}$ Alkyl, $C_2-C_5$ Alkenyl, Phenyl, $C_1-C_{12}$ Alkoxy, Phenoxy, $-NH-Alkyl-C_1-C_{12}$, $-NH$Phenyl oder $-NH$Cyclohexyl bedeutet und $R^6$ $C_4-C_{12}$ Alkyl oder $C_6-C_{10}$ Aryl bedeutet und

X im Falle von n = 2 eine direkte Bindung, $C_1-C_6$ Alkylen, Vinylen, Phenylen oder eine der zweiwertigen Gruppen $-CH_2S-SCH_2-$, $-CH(R^2)-O-CO-O-CH(R^2)-$, $-CH(R^2)-O-CO-R^7-CO-OCH(R^2)-$, $-CO-O-R^8-O-CO-$, $-CH_2COO-R^8-OOCCH_2-$ oder $-CH(OH)-CH_2-O-R^8-O-CH_2-CH(OH)-$ darstellt, worin $R^2$ die oben angegebene Bedeutung hat, $R^7$ $C_2-C_{10}$ Alkylen, Vinylen, Phenylen oder einen Rest $-NH-R^9-NH-$ darstellt, $R^8$ $C_2-C_8$ Alkylen, Phenylen oder $-$Phenylen$-C(CH_3)_2-$Phenylen bedeutet und $R^9$ $C_4-C_{10}$ Alkylen, $C_6-C_{12}$ Arylen, Tolylen oder $-$Phenylen$-CH_2-$Phenylen$-$ bedeutet, und

B) einem organischen Amin

als Initiator für die Photopolymerisation äthylenisch ungesättigter Verbindungen oder für die photochemische Vernetzung von Polyolefinen.

6. Verwendung eines Gemisches gemäß Anspruch 5, bestehend aus

A) einem Mercaptophenylketon der Formel I, worin
n 1 ist, Ar Phenyl, 2-Furyl, 2-Thienyl, 2-Pyridyl oder durch $C_1-C_4$ Alkyl, $C_1-C_4$ Alkoxy, $-COO-C_1-C_4$ Alkyl, $-SCH_2X$ oder Benzoyl substituiertes Phenyl darstellt, $R^1$ Wasserstoff ist und
X eine der Gruppen $-CH_2OH$, $-CH(CH_3)-OH$, $-CH(OH)-CH_2OH$, $-CH_2-NR^3R^4$ oder $-CH-O-CO-R^5$ darstellt, worin $R^3$ und $R^4$ $C_1-C_4$ Alkyl oder Hydroxyäthyl sind und $R^5$ $C_1-C_4$ Alkyl, Phenyl, Vinyl oder Propenyl ist, und

B) einem aliphatischen tertiären Amin, einem p-Dimethylaminobenzoesäurealkylester oder Michlers Keton (4,4'-Bis-dimethylamino-benzophenon).

7. Verwendung gemäß Anspruch 5 eines Gemisches aus

A) einem Mercaptophenylketon der Formel I, worin n = 1 ist, Ar Phenyl, Tolyl oder Xylyl ist, $R^1$ Wasserstoff ist und X $-CH_2OH$, $-CH_2NR^3R^4$ oder $-CH_2O-CO-R^5$ darstellt, worin $R^3$ und $R^4$ Methyl, Äthyl oder zusammen 1,5-Pentylen bedeuten und $R^5$ $C_1-C_4$ Alkyl oder Phenyl bedeutet, und

B) Triäthanolamin oder einem $C_1-C_4$ Alkyl-di-äthanolamin.

8. Verwendung gemäß Anspruch 5 eines Gemisches aus

A) 4-(2-Hydroxyäthyl-mercapto)-benzophenon oder einem Carbonsäureester davon und

B) einem aliphatischen tertiären Amin, einem p-Dimethylaminobenzoesäurealkylester oder Michlers Keton.

9. Verwendung eines Gemisches gemäß Anspruch 5, mit einer Zusammensetzung von 4 Gew.-Teilen der Komponente A und 1 Gew.-Teil der Komponente B bis zu 1 Gew.-Teil der Komponente A und 4 Gew.-Teile der Komponente B.

10. Verwendung eines Gemisches gemäß Anspruch 5 als Initiator für die Photopolymerisation von Acrylsäureestern und deren Gemischen.

11. Verwendung eines Gemisches gemäß Anspruch 5 als Initiator für die Photohärtung von Druckfarben.

12. Photopolymerisierbares System, bestehend aus a) mindestens einer äthylenisch ungesättigten Verbindung, b) einem Gemisch gemäß einem der Ansprüche 5—8 als Initiator und gegebenenfalls c) sonstigen Zusatzstoffen wie Inhibitoren, Stabilisatoren, UV-Absorbern, Füllstoffen, Pigmenten, Farbstoffen, Thixotropiemittel oder Verlaufshilfsmittel.

13. Photopolymerisierbares System gemäß Anspruch 12 in den Mengenverhältnissen 99,5 bis 80 Gew.-% der Summe von a und c und 0,5 bis 20 Gew.-% von b.

14. Photopolymerisierbares System gemäß Anspruch 12, worin die Komponente a ein Acrylsäureester oder Gemisch mehrerer Acrylsäureester ist.

**Claims**

1. A compound of the formula I

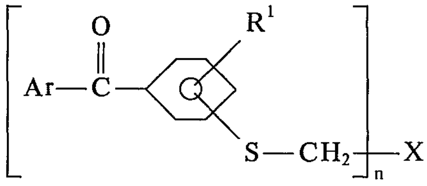

(I)

wherein

n is 1 or 2,

Ar is a $C_6-C_{10}$aryl radical, which can be unsubstituted or substituted by one or more of the groups $C_1-C_4$alkyl, phenyl, $C_1-C_4$alkoxy, phenoxy, $C_5-C_6$cycloalkoxy, halogen, $-COOH$, $-COO-(C_1-C_4$alkyl) or benzoyl or, if n is 1, also by $-SCH_2X$, or is a 5-membered or 6-membered hetero-aromatic radical,

$R^1$ is hydrogen, $C_1-C_4$alkyl or, if n is 1, also $-SCH_2X$ and

X if n is 1, is one of the groups $-CH(R^2)-OH$, $-CH(R^2-O-CO-R^5$, $-CH_2SH$ or $-(CH_2)_{1-3}-NR^3R^4$, in which $R^2$ is hydrogen, methyl, phenyl or one of the groups $-CH_2OH$, $-CH_2NR^3R^4$ or $-CH_2-OR^6$, $R^3$ and $R^4$ independently of each other are hydrogen, $C_1-C_8$alkyl or $C_2-C_4$hydroxyalkyl, or $R^3$ and $R^4$ together are 1.4-butylene, 1.5-pentylene or 3-oxa-1.5-pentylene, $R^5$ is $C_1-C_{12}$alkyl, $C_2-C_5$alkenyl, phenyl, $C_1-C_{12}$alkoxy, phenoxy, $-NH-(C_1-C_{12}$alkyl), $-NH-$phenyl or $-NH-$cyclohexyl and $R^6$ is $C_4-C_{12}$alkyl or $C_6-C_{10}$aryl, and if n is 2, X is a direct bond, $C_1-C_6$alkylene, vinylene, phenylene or one of the divalent groups $-CH_2S-SCH_2-$, $-CH(R^2)-O-CO-O-CH(R^2)-$, $-CH(R^2)-O-CO-R^7-CO-OCH(R^2)-$, $-CO-O-R^8-O-CO-$, $-CH_2COO-R^8-OOCCH_2-$ or $-CH(OH)-CH_2-O-R^8-O-CH_2-CH(OH)-$, in which $R^2$ is as defined above, $R^7$ is $C_2-C_{10}$alkylene, vinylene, phenylene or a radical $-NH-R^9-NH-$, $R^8$ is $C_2-C_8$alkylene, phenylene or $-$phenylene$-C(CH_3)_2-$phenylene and $R^9$ is $C_4-C_{10}$alkylene, $C_6-C_{12}$arylene, tolylene or $-$phenylene$-CH_2-$phenylene.

2. A compound according to claim 1 of the formula I, wherein n is 1, Ar is phenyl, 2-furyl, 2-thienyl, 2-pyridyl or phenyl which is substituted by $C_1-C_4$alkyl, $C_1-C_4$alkoxy, $-COO-(C_1-C_4$alkyl), $-SCH_2X$ or benzoyl, $R^1$ is hydrogen and X is one of the groups $-CH_2OH$, $-CH(CH_3)-OH$, $-CH(OH)-CH_2OH$, $-CH_2-NR^3R^4$ or $-CH-O-CO-R^5$, in which $R^3$ and $R^4$ are $C_1-C_4$alkyl or hydroxyethyl and $R^5$ is $C_1-C_4$alkyl, phenyl, vinyl or propenyl.

3. A compound according to claim 1 of the formula I, wherein n is 1, Ar is phenyl, tolyl or xylyl, $R^1$ is hydrogen and X is $-CH_2OH$, $-CH_2NR^3R^4$ or $-CH_2O-CO-R^5$, in which $R^3$ and $R^4$ are methyl or ethyl or together are 1.5-pentylene and $R^5$ is $C_1-C_4$alkyl or phenyl.

4. A compound according to claim 1 which is 4-(2-hydroxyethylmercapto)benzophenone, or a carboxylic acid ester thereof.

5. Use of a mixture of

A) a mercaptophenylketone of the formula I

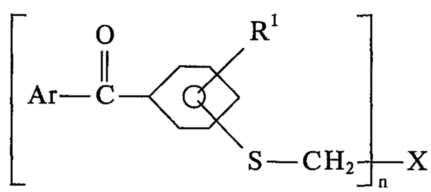

(I)

wherein

n is 1 or 2,

Ar is a $C_6-C_{10}$aryl radical, which can be unsubstituted or substituted by one or more of the groups $C_1-C_4$alkyl, phenyl, $C_1-C_4$alkoxy, phenoxy, $C_5-C_6$cycloalkoxy, halogen, $-COOH$, $-COO-(C_1-C_4$alkyl) or benzoyl or, if n is 1, also by $-SCH_2X$, or is a 5-membered or 6-membered hetero-aromatic radical,

$R^1$ is hydrogen, $C_1-C_4$alkyl or, if n is 1, also $-SCH_2X$ and

X if n is 1, is one of the groups $-CH(R^2)-OH$, $-CH(R^2-O-CO-R^5$, $-CH_2SH$ or $-(CH_2)_{1-3}-NR^3R^4$, in which $R^2$ is hydrogen, methyl, phenyl or one of the groups $-CH_2OH$, $-CH_2NR^3R^4$ or $-CH_2-OR^6$, $R^3$ and $R^4$ independently of each other are hydrogen, $C_1-C_8$alkyl

or $C_2-C_4$hydroxyalkyl, or $R^3$ an $R^4$ together are 1.4-butylene, 1.5-pentylene or 3-oxa-1.5-pentylene, $R^5$ is $C_1-C_{12}$alkyl, $C_2-C_5$alkenyl, phenyl, $C_1-C_{12}$alkoxy, phenoxy, $-NH-(C_1-C_{12}alkyl)$, $-NH-$phenyl or $-NH-$cyclohexyl and $R^6$ is $C_4-C_{12}$alkyl or $C_6-C_{10}$aryl, and if n is 2, X is a direct bond, $C_1-C_6$alkylene, vinylene, phenylene or one of the divalent groups $-CH_2S-SCH_2-$, $-CH(R^2)-O-CO-O-CH(R^2)-$, $-CH(R^2)-O-CO-R^7-CO-OCH(R^2)-$, $-CO-O-R^8-O-CO-$, $-CH_2COO-R^8-OOCCH_2-$ or $-CH(OH)-CH_2-O-R^8-O-CH_2-CH(OH)-$, in which $R^2$ is as defined above, $R^7$ is $C_2-C_{10}$alkylene, vinylene, phenylene or a radical $-NH-R^9-NH-$, $R^8$ is $C_2-C_8$alkylene, phenylene or $-$phenylene$-C(CH_3)_2-$phenylene and $R^9$ is $C_4-C_{10}$alkylene, $C_6-C_{12}$arylene, tolyleneor$-$phenylene$-CH_2-$phenylene and

B) an organic amine,

as initiator for the photopolymerisation of ethylenically unsaturated compounds or for the chemical crosslinking of polyolefins.

6. Use of a mixture according to claim 5, consisting of

A) a mercaptophenylketone of the formula I, wherein n is 1, Ar is phenyl, 2-furyl, 2-thienyl, 2-pyridyl or phenyl which is substituted by $C_1-C_4$alkyl, $C_1-C_4$alkoxy, $-COO-(C_1-C_4alkyl)$, $-SCH_2X$ or benzoyl, $R^1$ is hydrogen and X is one of the groups $-CH_2OH$, $-CH(CH_3)-OH$, $-CH(OH)-CH_2OH$, $-CH_2-NR^3R^4$ or $-CH-O-CO-R^5$, in which $R^3$ and $R^4$ are $C_1-C_4$alkyl or hydroxyethyl and $R^5$ is $C_1-C_4$alkyl, phenyl, vinyl or propenyl, and

B) an aliphatic tertiary amine, a p-dimethylaminobenzoic acid alkyl ester or Michler's ketone (4.4'-bis-dimethylaminobenzophenone).

7. Use according to claim 5 of a mixture of

A) a mercaptophenylketone of the formula I, wherein n is 1, Ar is phenyl, tolyl or xylyl, $R^1$ is hydrogen and X is $-CH_2OH$, $-CH_2NR^3R^4$ or $-CH_2O-CO-R^5$, in which $R^3$ and $R^4$ are methyl or ethyl or together are 1.5-pentylene and $R^5$ is $C_1-C_4$alkyl or phenyl, and

B) triethanolamine or a $C_1-C_4$alkyldiethanolamine.

8. Use according to claim 5 of a mixture of

A) 4-(2-hydroxyethylmercapto)benzophenone or a carboxylic acid ester thereof, and

B) an aliphatic tertiary amine, a p-dimethylaminobenzoic acid alkyl ester or Michler's ketone.

9. Use of a mixture according to claim 5 having a composition of 4 parts by weight of component A and 1 part by weight of component B to 1 part by weight of component A and 4 parts by weight of component B.

10. Use of a mixture according to claim 5 as initiator for the photopolymerisation of acrylic acid esters and mixtures thereof.

11. Use of a mixture according to claim 5 as initiator for photohardening printing inks.

12. A photopolymerisable system consisting of a) at least one ethylenically unsaturated compound, b) a mixture of a compound according to any one of claims 5 to 8 as photoinitiator and, if desired, c) other additives such as inhibitors, stabilisers, UV absorbers, fillers, pigments, dyes, thixotropic agents of flow control agents.

13. A photopolymerisable system according to claim 12 in ratios of 99.5 to 80% by weight of the sum of a and c to 0.5 to 20% by weight of b.

14. A photopolymerisable system according to claim 12, in which component a is an acrylic acid ester or a mixture of several acrylic acid esters.

**Revendications**

1. Composé répondant à la formule I

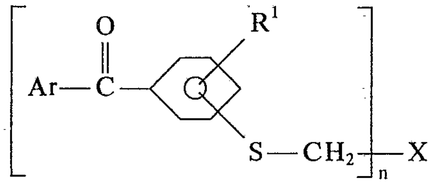

(I)

dans laquelle

n est égal à 1 ou à 2
Ar représente un aryle en $C_6-C_{10}$ non substitué ou porteur d'un ou de plusieurs substituants pris dans l'ensemble constitué par les alkyles en $C_1-C_4$, le phényle, les alcoxy en $C_1-C_4$, le phénoxy, les cycloalcoxy en $C_5$ et $C_6$, les halogènes, $-COOH$, COO-Alkyl$-C_1-C_4$, le benzoyle, et, lorsque n est égal à 1, également les radicaux $-SCH_2X$, ou représente un radical hétéro-aromatique à 5 ou 6 maillons,
$R^1$ représente l'hydrogène, un alkyle en $C_1-C_4$ ou, lorsque n est égal à 1, également un radical $-SCH_2X$, et
X représente, dans le cas où n est égal à 1, l'un des radicaux $-CH(R^2)-OH$, $-CH(R^2)-O-CO-R^5$, $-CH_2SH$ ou $-(CH_2)_{1-3}-NR^3R^4$, dans lesquels $R^2$ représente l'hydrogène, un méthyle, un phényle ou l'un des radicaux $-CH_2OH$, $-CH_2-NR^3R^4$ et $-CH_2-OR^6$, $R^3$ et $R^4$ représentent chacun, indépendamment l'un de l'autre, l'hydrogène, un alkyle en $C_1-C_8$ ou un hydroxy-alkyle en $C_2-C_4$, ou encore $R^3$ et $R^4$ forment ensemble un radical butylène-1,4, pentylène-1,5 ou oxa-3-pentylène-1,5, $R^5$ représente un alkyle en $C_1-C_{12}$, un alcényle en $C_2-C_5$, un phényle, un alcoxy en $C_1-C_{12}$, un phénoxy, ou un radical $-NH-$alkyl$-C_1-C_{12}$, $-$NHPhényl ou $-$NHCyclohexyl, et $R^6$ représente un alkyle en $C_4-C_{12}$ ou un aryle en $C_6-C_{10}$, et
X représente, dans le cas où n est égal à 2, une liaison directe un alkylène en $C_1-C_6$, un vinylène, un phénylène ou l'un des radicaux bivalents suivants: $-CH_2S-SCH_2-$, $-CH(R^2)-O-CO-O-CH(R^2)-$, $-CH(R^2)-O-CO-R^7-CO-OCH(R^2)-$, $-CO-O-R^8-O-CO-$, $-CH_2COO-R^8-OOCCH_2-$ et $-CH(OH)-CH_2-O-R^8-O-CH_2-CH(OH)-$, où $R^2$ à la signification précédemment donnée, $R^7$ représente un alkylène en $C_2-C_{10}$, un vinylène, un phénylène ou un radical $-NH-R^9-NH-$, $R^8$ représente un alkylène en $C_2-C_8$, un phénylène ou un radical phénylène$-C(CH_3)_2-$phénylène$-$ et $R^9$ représente un alkylène en $C_4-C_{10}$, un arylène en $C_6-C_{12}$, un toluène ou un radical $-$phénylène$-CH_2-$phénylène$-$.

2. Composé de formule I selon la revendication 1, dans lequel:

n est égal à 1,
Ar représente un radical phényle, furyle-2, thiényle-2, pyridyle-2, ou un radical phényle porteur d'un alkyle en $C_1-C_4$, d'un alcoxy en $C_1-C_4$, d'un radical $-COO-C_1-C_4-$ alkyl, d'un radical $-SCH_2X$ ou d'un radical benzoyle,
$R^1$ représente l'hydrogène, et
X représente l'un des radicaux $-CH_2OH$, $-CH(CH_3)-OH$, $-CH(OH)-CH_2-OH$, $-CH_2-NR^3R^4$ et $-CH_2-O-CO-R^5$, où $R^3$ et $R^4$ désignent un alkyle en $C_1-C_4$ ou un hydroxy-éthyle et $R^5$ désigne un alkyle en $C_1-C_4$, un phényle, un vinyle ou un propényle.

3. Composé de formule I selon la revendication 1, dans lequel:

n est égal à 1,
Ar représente un radical phényle, tolyle ou xylyle,
$R^1$ représente l'hydrogène et
X représente $-CH_2OH$, $-CH_2NR^3R^4$ ou $-CH_2O-CO-R^5$, les symboles $R^3$ et $R^4$ représentant chacun un radical méthyle ou un radical éthyle ou formant ensemble un radical pentylène-1,5 et $R^5$ représentant un alkyle en $C_1-C_4$ ou un phényle.

4. Composé selon la revendication 1, en l'espèce l'(hydroxy-2 éthylthio)-4 benzophénone ou l'un des ses esters d'acides carboxyliques.

5. Application d'un mélange constitué:

A) d'une mercaptophénylcétone répondant à la formule I

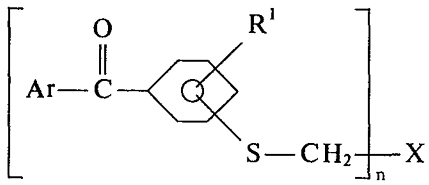

(I)

dans laquelle

n est égal à 1 ou à 2
Ar représente un aryle en $C_6-C_{10}$ non substitué ou porteur d'un ou de plusieurs substituants pris dans l'ensemble constitué par les alkyles en $C_1-C_4$, le phényle, les alcoxy en $C_1-C_4$, le phénoxy, les cycloalcoxy en $C_5$ et $C_6$, les halogènes, $-COOH$, COO−Alkyl−$C_1-C_4$, le benzoyle, et, lorsque n est égal à 1, également les radicaux $-SCH_2X$, ou représente un radical hétéro-aromatique à 5 ou 6 maillons,
$R^1$ représente l'hydrogène, un alkyle en $C_1-C_4$ ou, lorsque n est égal à 1, également un radical $-SCH_2X$, et
X représente, dans le cas où n est égal à 1, l'un des radicaux $-CH(R^2)-OH$, $-CH(R^2)-O-CO-R^5$, $-CH_2SH$ ou $-(CH_2)_{1-3}-NR^3R^4$, dans lesquels $R^2$ représente l'hydrogène, un méthyle, un phényle ou l'un des radicaux $-CH_2OH$, $-CH_2-NR^3R^4$ et $-CH_2-OR^6$, $R^3$ et $R^4$ représentent chacun, indépendamment l'un de l'autre, l'hydrogène, un alkyle en $C_1-C_8$ ou un hydroxy-alkyle en $C_2-C_4$, ou encore $R^3$ et $R^4$ forment ensemble un radical butylène-1,4, pentylène-1,5 ou oxa-3-pentylène-1,5, $R^5$ représente un alkyle en $C_1-C_{12}$, un alcényle en $C_2-C_5$, un phényle, un alcoxy en $C_1-C_{12}$, un phénoxy, ou un radical −NH−alkyl−$C_1-C_{12}$, −NHPhényl ou −NHCyclohexyle, et $R^6$ représente un alkyle en $C_4-C_{12}$ ou un aryle en $C_6-C_{10}$,

et, dans le cas où n est égal à 2, une liaison directe, un alkylène en $C_1-C_6$, un vinylène, un phénylène ou l'un des radicaux bivalents suivants: $-CH_2S-SCH_2-$, $-CH(R^2)-O-CO-O-CH(R^2)-$, $-CH(R^2)-O-CO-R^7-CO-OCH(R^2)-$, $-CO-O-R^8-O-CO-$, $-CH_2COO-R^8-OOCCH_2-$ et $-CH(OH)-CH_2-O-R^8-O-CH_2-CH(OH)-$, où $R^2$ à la signification précédemment donnée, $R^7$ représente un alkylène en $C_2-C_{10}$, un vinylène, un phénylène ou un radical $-NH-R^9-NH-$, $R^8$ représente un alkylène en $C_2-C_8$, un phénylène ou un radical −phénylène−$C(CH_3)_2$−phénylène− et $R^9$ représente un alkylène en $C_4-C_{10}$, un arylène en $C_6-C_{12}$, un toluène ou un radical −phénylène−$CH_2$−phénylène−, et
B) d'une amine organique,

comme amorceur pour la photopolymérisation de composés éthyléniques ou pour la réticulation photo-chimique de polyoléfines.

6. Application d'un mélange selon la revendication 5, constitué:

A) d'une mercaptophénylcétone de formule I dans laquelle n est égal à 1, Ar représente un radical phényle, furyle-2, thiényle-2, pyridyle-2, ou un radical phényle porteur d'un alkyle en $C_1-C_4$, d'un alcoxy en $C_1-C_4$, d'un radical $-COO-C_1-C_4-$ alkyle, d'un radical $-SCH_2X$ ou d'un radical benzoyle, $R^1$ représente l'hydrogène, et X représente l'un des radicaux $-CH_2OH$, $-CH(CH_3)-OH$, $-CH(OH)-CH_2-OH$, $-CH_2-NR^3R^4$ et $-CH_2-O-CO-R^5$, où $R^3$ et $R^4$ désignent un alkyle en $C_1-C_4$ ou un hydroxy-éthyle et $R^5$ désigne un alkyle en $C_1-C_4$, un phényle, un vinyle ou un propényle, et
B) d'une amine tertiaire aliphatique, d'un p-diméthylamino-benzoate d'alkyle ou de la cétone de Michler (bis-diméthylamino-4,4' benzophénone).

7. Application selon la revendication 5 d'un mélange constitué:

A) d'une mercaptophénylcétone de formule I dans laquelle n est égal à 1, Ar représente un radical phényle, tolyle ou xylyle, $R^1$ l'hydrogène et X l'un des radicaux $-CH_2OH$, $-CH_2NR^3R^4$ et $-CH_2O-CO-R^5$ dans lesquels $R^3$ et $R^4$ représentent chacun un méthyle ou un éthyle ou forment ensemble un pentylène-1,5 et $R^5$ représente un alkyle en $C_1-C_4$ ou un phényle, et
B) de triéthanolamine ou d'une alkyl-diéthanolamine à alkyle en $C_1-C_4$.

8. Application selon la revendication 5 d'un mélange constitué:

A) d'(hydroxy-2 éthylthio)-4 benzophénone ou d'un ester d'acide carboxylique de celle-ci et

B) d'une amine tertiaire aliphatique, d'un p-diméthylamino-benzoate d'alkyle ou de cétone de Michlers.

9. Application d'un mélange selon la revendication 5 dans lequel le rapport pondéral de la composante A à la composante B est compris entre 4 : 1 et 1 : 4.

10. Application d'un mélange selon la revendication 5 comme amorceur pour la photopolymérisation d'esters acryliques et de leurs mélanges.

11. Application d'un mélange selon la revendication 5 comme amorceur pour le photo-durcissement d'encres d'imprimerie.

12. Système photopolymérisable constitué a) d'au moins un composé éthylénique, b) d'un mélange selon l'une des revendications 5 à 8 en tant qu'amorceur, et, éventuellement, c) d'autres additifs, tels que des inhibiteurs, des stabilisants, des absorbeurs de rayons ultra-violets, des charges, des pigments, des colorants, des agents de thixotropie et des agents de nivellement.

13. Système photopolymérisable selon la revendication 12, dans lequel la somme des constituants a) et c) représente de 99,5 à 80% en poids et les constituants b) représentent de 0,5 à 20% en poids.

14. Système photopolymérisable selon la revendication 12 dans lequel la composante a) est un ester de l'acide acrylique ou un mélange du plusieurs esters de l'acide acrylique.